# EUROPÄISCHE PATENTSCHRIFT

(11) **EP 1 099 468 B1**
(45) Veröffentlichungstag und Bekanntmachung des Hinweises auf die Patenterteilung: **25.07.2007**
(21) Anmeldenummer: 00124187.6
(22) Anmeldetag: 08.11.2000
(51) Int. Cl.: B01D 71/52, B01D 71/68, B01D 71/82, B01D 67/00, A61F 2/02, A61L 27/38, A61K 9/00

(54) **Mikroporöse hydrophile Membran**
Microporous hydrophilic membrane
Membrane microporeuse hydrophile

(30) Priorität: 10.11.1999 DE 19954158
(43) Veröffentlichungstag der Anmeldung: 16.05.2001
(73) Patentinhaber: DEUTSCHE INSTITUTE FÜR TEXTIL- UND FASERFORSCHUNG STUTTGART Stiftung des öffentlichen Rechts, 73770 Denkendorf (DE)
(72) Erfinder: Zschocke, Peter, Dr., 72116 Mössingen (DE); Enderle, Anja, 72654 Neckartenzlingen (DE); Doser, Michael, Dr., 70794 Filderstadt (DE); Planck, Heinrich, Prof. Dr., 72622 Nürtingen (DE)
(74) Vertreter: Patentanwälte Ruff, Wilhelm, Beier, Dauster & Partner

(56) Entgegenhaltungen:
- EP-A- 0 331 298
- DE-A- 19 622 338
- US-A- 4 797 457
- US-A- 4 894 159
- US-A- 5 173 415
- US-A- 5 376 689
- US-A- 5 693 740
- US-A- 5 837 234
- MOCKEL D ET AL: "Static protein adsorption, ultrafiltration behavior and cleanability of hydrophilized polysulfone membranes" JOURNAL OF MEMBRANE SCIENCE, ELSEVIER SCIENCE, AMSTERDAM, NL, Bd. 158, Nr. 1-2, 1. Juni 1999 (1999-06-01), Seiten 63-75, XP004166227 ISSN: 0376-7388
- PATENT ABSTRACTS OF JAPAN vol. 012, no. 172 (C-497), 21. Mai 1988 (1988-05-21) -& JP 62 279805 A (TORAY IND INC), 4. Dezember 1987 (1987-12-04) -& DATABASE WPI Derwent Publications Ltd., London, GB; AN 1988-017776 XP002191685 & JP 62 279805 A

## Beschreibung

Die Erfindung betrifft mikroporöse, hydrophile Membranen, welche für niedermolekulare Substanzen, insbesondere Proteine, durchlässig sind. Weiterhin betrifft die Erfindung Verfahren zur Herstellung derartiger Membranen.

In vielen Bereichen der Biotechnologie, Lebensmittelindustrie und insbesondere der Biomedizin ist es von außerordentlichem Interesse, bestimmte Makromoleküle wie beispielsweise Enzyme, Antikörper oder bestimmte Hormone oder Signalstoffe von anderen Substanzen abzutrennen. Dies spielt beispielsweise bei der Proteinreinigung von gentechnisch hergestellten Wirkstoffen eine Rolle. Auch im Bereich der Dialyse von Nierenpatienten oder bei der Herstellung künstlicher Kapillaren sind die Trennungseigenschaften der eingesetzten Membranen von au-ßerordentlicher Bedeutung.

Gewöhnlicherweise werden für die Membranen, mit deren Hilfe makromolekulare Gemische aufgetrennt werden sollen, mikroporöse Membranen auf Polymerbasis eingesetzt. In der Regel weisen solche Membranen eine asymmetrische Struktur auf.

Die Oberseite einer solchen Membran kann als dünne, feinporöse, 0,2 bis 2 µm dicke Schicht, die eigentliche Membran, ausgebildet sein. Diese Membran kann von einer beispielsweise etwa 50 bis etwa 200 µm dicken Unterstruktur unterstützt sein, welche nach unten hin zunehmend grobporiger aufgebaut ist.

Hergestellt werden solche asymmetrischen Membranen vornehmlich nach einem von Loeb und Sourirajan entwickelten, als Phaseninversion bezeichneten Verfahren. Dabei wird ein Polymer in einem Lösungsmittel gelöst, als Film ausgebreitet und mit einem Nichtlösungsmittel, dem sog. Fällmittel, zu einer Phaseninversionsmembran gefällt. Das Fällmittel ist mit dem Polymerlösungsmittel unbegrenzt mischbar. Daher wird das Lösungsmittel durch das Fällmittel immer mehr verdünnt, bis das Polymer als Membran ausfällt.

Nach diesem Verfahren können aus verschiedenen löslichen Polymeren asymmetrisch strukturierte Membranen hergestellt werden. Beispiele für geeignete Polymere sind Celluloseacetate, Polyamide, Polyolefine, Polysulfone und Polyetherketone. Je nach eingesetztem Fällmittel bildet sich eine bestimmte Struktur der Membran aus. Fällmittel, die sich mit hoher Mischungswärme im Polymerlösungsmittel lösen, führen zur Ausformung einer fingerstrukturierten Membran. Fällmittel mit geringer Mischungswärme führen hingegen zu schwammartig strukturierten Membranen. Durch die Wahl des Fällmittels bzw. auch des Lösungsmittels ist also die Struktur einer Membran einstellbar.

Der Trennmechanismus dieser Membranen beruht u.a. auf dem Ausschluß aller Makromoleküle, die größere Moleküldurchmesser haben als die Porendurchmesser der Membranoberseite. Makromoleküle mit deutlich kleineren Moleküldurchmessern können prinzipiell die Membran permeieren. Diese molekulare Trenngrenze oder Ausschlußgrenze wird so definiert, daß 90 % eines Testmoleküls bekannter Molekülgröße von der Membran zurückgehalten wird. Durch entsprechende Wahl der verwendeten Polymere und der Bedingungen der Membranherstellung kann eine bestimmte molekulare Trenngrenze hergestellt werden.

Bei reiner Diffusion ist die treibende Kraft für den Durchtritt der Moleküle, also die Permeation, der transmembran wirkende osmotische Druckgradient der jeweiligen Moleküle bzw. der Permeanten.

Die bisher üblichen Diffusionsmembranen sind in der Regel hydrophob. Dies ist vor allem bei der diffusiven Trennung von Proteinen unterschiedlicher Molekülgrößen sehr nachteilig, da die Proteine an den Membranoberflächen und an der Membranmatrix anhaften. Dies führt zum einen zur Ausbildung von Deckschichten (fouling) auf der Membranoberfläche, wodurch die Membraneigenschaften erheblich verändert werden. Weiterhin werden die Poren der Membran durch die Adsorption von Proteinen verkleinert bzw. vollständig verstopft. Hierdurch wird die Permeatleistung erheblich reduziert oder vollständig unterbunden. Die Funktionsfähigkeit solcher Membranen ist also sehr eingeschränkt und nur von kurzer Dauer. Prinzipiell sind solche Membranen für die diffusive Trennung von Proteinen also ungeeignet.

Es wurden verschiedene Versuche unternommen, ein hydrophileres Material für Membranen zu verwenden, um diese negativen adsorptiven Eigenschaften von herkömmlichen Membranen zu umgehen. Membranen, die auf einem hydrophilen Material wie beispielsweise Celluloseacetat basieren, weisen jedoch nicht die notwendige Temperaturstabilität auf und sind empfindlich gegenüber chemischen oder mikrobiologischen Agenzien. Derartige hydrophile Membranmaterialien stellen also auch keine Lösung des Problems dar.

Als ein Lösungsweg wurde vorgeschlagen, die Matrix von hydrophoben Membranen durch die Adsorption von hydrophilen Komponenten zu modifizieren, um so eine Membran mit antiadsorptiven Eigenschaften bereitstellen zu können. Hierdurch wurde erreicht, daß die Proteine in Lösung nicht adsorbiert wurden, sondern vielmehr von der Membran zurückgewiesen wurden und in Lösung blieben. Somit wurde die Ausbildung von Deckschichten auf der Membranoberfläche vermieden. Andererseits konnten bezüglich der Durchgängigkeit der Membranen für Proteine keine befriedigenden Ergebnisse erzielt werden, so daß nach wie vor das Bedürfnis nach einer gut funktionierenden und langlebigen antiadsorptiven Membran besteht.

Aus der Veröffentlichung von Möckel, Staude, Guiver; "Static protein absorption, ultrafiltration behavior and cleanability of hydrophilized polysulfone membranes", Journal of Membrane Science, Elsevier Science, Amsterdam, NL, Bd. 158, Nr. 1 - 2, vom 1. Juni 1999 sind hydrophile Membranen bekannt, die aus mit Carboxylgruppen substituierten Polysulfonen hergestellt sind. Solche Membranen zeigen bereits gute Trenneigenschaften bei niedermolekularen Proteinen. Bei geringen Proteinmengen ist die Adsorption an der Membran jedoch noch zu groß. Die Herstellung ähnlicher Membranen geht aus der DE 196 22 338 A1 hervor, wobei eine asymetrische Porosität bei der Membranherstellung durch Phasenumkehr erreicht wird.

Die US 4,797,457 beschreibt allgemein die Substituierung von Polysulfonen mit unterschiedlichsten Substituenten und ihre Eignung als Membranen. Es ist dort auch die Herstellung der vorgehend erwähnten mit Carboxylgruppen substituierten Polysulfone beschrieben. Auch wird erwähnt, dass die Umsetzung von mit Lithium substituierten Polysulfonen mit Aldehyden und Ketonen zu Polymeren mit hydrophilen Eigenschaften führt. Für die Probleme im Zusammenhang mit der Diffusion geringer Proteinmengen finden sich dort jedoch keine Hinweise.

In der US 5,837,234 sind bioartifizielle Organe beschrieben, bei denen lebende Zelle in hydrophilen Polyethersulfon-Membranen enthalten sind. Das Polyethersulfon-Membranmaterial kann durch Coextrusion durch eine Ringdüse in Rohrform hergestellt werden und hat feinporige innere und äußere Oberflächen, die durch eine offenporige Stützstruktur verbunden sind.

Die Erfindung stellt sich die Aufgabe, eine Membran bereitzustellen, die in besonderer Weise antiadsorptiv für Proteine ist und so die geschilderten Nachteile des Standes der Technik vermeidet. Die Membran soll insbesondere als Diffusionsmembran für kleine Proteinmengen und für biohybride Organe geeignet sein. Die Aufgabe wird gelöst durch eine mikroporöse, hydrophile Membran mit den im Anspruch 1 beschriebenen Merkmalen. Bevorzugte Ausführungsformen dieser Membran sind in den Ansprüchen 2 bis 4 beschrieben. Ein Verfahren zur Herstellung einer solchen mikroporösen, hydrophilen Membran ist in Anspruch 5 beansprucht. Bevorzugte Ausführungsformen des Verfahrens sind in den Ansprüchen 6 bis 9 dargestellt. Die Ansprüche 10 bis 15 betreffen im wesentlichen die Verwendung einer er-findungsgemäßen Membran, einschliesslich eines biohybriden Hohlorgans (Anspruch 13). Der Wortlaut sämtlicher Ansprüche wird hiermit durch Bezugnahme zum Inhalt der Beschreibung gemacht.

Gegenstand der Erfindung ist eine mikroporöse, hydrophile Membran, in Form einer Kapillare aus chemisch substituiertem Polysulfon, welches kovalent gebundene Methylenhydroxylgruppen aufweist, wobei jede sich wiederholende Polymereinheit einen Substitutionsrad (DS) von 0,5 bis 1,9 Methylenhydroxylgruppen besitzt, die Membran eine Stärke von 20 µm bis 400 µm aufweist, eine Porosität, bezogen auf das Volumen, von 50 % bis 90 % und im Querschnitt eine im wesentlichen zunehmende Porengröße aufweist, wobei die Porengröße im Bereich der kleineren Poren zwischen 10 nm und 50 nm beträgt, die Membran eine molekulare Trenngrenze von 5.000 Dalton bis 100.000 Dalton aufweist und in sterilem, feuchtem Zustand verpackt ist.

Durch die Modifikation der an sich hydrophoben Polymere, die chemisch hochstabil und biokompatibel sind, wird erreicht, daß eine aus diesen substituierten Polymeren hergestellte Membran hydrophile und damit antiadsorptive Eigenschaften besitzt. Einerseits wird also erreicht, daß die erfindungsgemäße Membran die positiven Eigenschaften hinsichtlich Stabilität und Lebensdauer einer Membran aus hydrophoben Polymeren besitzt und andererseits führt die Modifizierung der Polymere zu einer Membran, die für beispielsweise eine diffusive Trennung von Proteinen unterschiedlicher Molekülgrößen in besonderer Weise geeignet ist. Im Gegensatz zu den herkömmlichen Oberflächen-Hydrophilisierungen von an sich hydrophoben Membranen weist die erfindungsgemäße Membran durchgängig eine hydrophile Struktur auf. Hierdurch wird eine Adsorption von Proteinen sowohl auf der Oberfläche als auch in den Poren bzw. an der Membranmatrix vermieden, so daß die oben beschriebenen Probleme der kurzen Lebensdauer und der Verstopfung der Poren von herkömmlichen Membranen bei der erfindungsgemäßen Membran nicht auftreten.

Auch bei einer langfristigen Benutzung nutzt sich die erfindungsgemäße Membran nicht ab, da sie massiv hydrophil ist. Überraschenderweise wird bei der erfindungsgemäßen Membran auch die Festigkeit der Membran nicht beeinträchtigt. Im allgemeinen wird davon ausgegangen, daß eine Membran aus hydrophilen Komponenten nicht die Stabilität einer hydrophoben Membran aufweist. Durch die Kombination von hydrophoben Basispolymeren mit hydrophilen Substituenten werden zum einen die positiven Eigenschaften, das heißt also Stabilität und Lebensdauer, einer hydrophoben Membran mit den ausgezeichneten antiadsorptiven Eigenschaften einer hydrophilen Membran miteinander kombiniert.

Als hydrophile Substituenten dienen Methylenhydroxylgruppen. Diese sind in besonderer Weise geeignet, da hier der Abstand zwischen hydrophiler OH-Gruppe und hydrophobem Basispolymer für viele Anwendungen optimal ist.

Vorzugsweise werden als Basispolymere aromatische Polysulfone eingesetzt. Beispiele für besonders geeignete Basispolymere sind Udel^{®}-Polysulfon (PSU), Radel^{®}-Polyphenylensulfon (PPSU), Victrex^{®}-Polyethersulfon (PES) Beispiele für Strukturformeln solcher Basispolymereinheiten sind in Schema a) dargestellt.

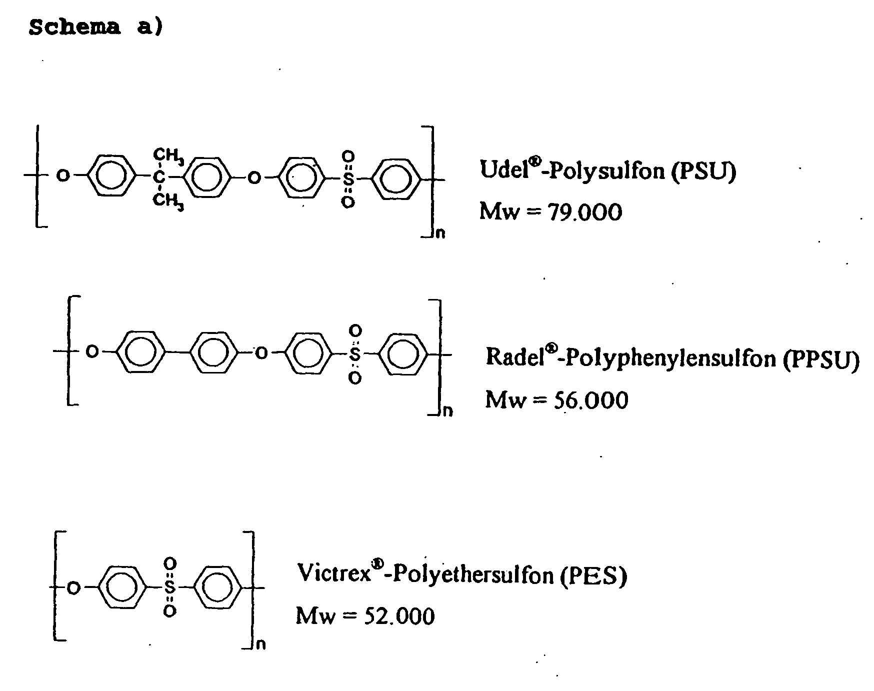

Die Modifikation dieser Basispolymere mit hydrophilen Substituenten erfolgt im wesentlichen nach bekannten Methoden (DE 36 36 854 A1). Der Substitutionsgrad (DS) beträgt 0,5 bis 1,9 pro wiederkehrende Einheit. Durch Variation der Verhältnisse während der Derivatisierung, z.B. der Reaktanden, Polysulfone: Butyllithium bzw. der Verweilzeit des PEEK in 98 %iger Schwefelsäure, können Polymer-Derivate verschiedener Substitutionsgrade synthetisiert werden. Oberhalb eines Substitutionsgrades von 2 kann unter Umständen die Stabilität der Membran beeinträchtigt sein bzw. eine unerwünschte Löslichkeit auftreten.

Die Modifikation der Basispolymere kann beispielsweise nach dem folgenden Schema ablaufen.

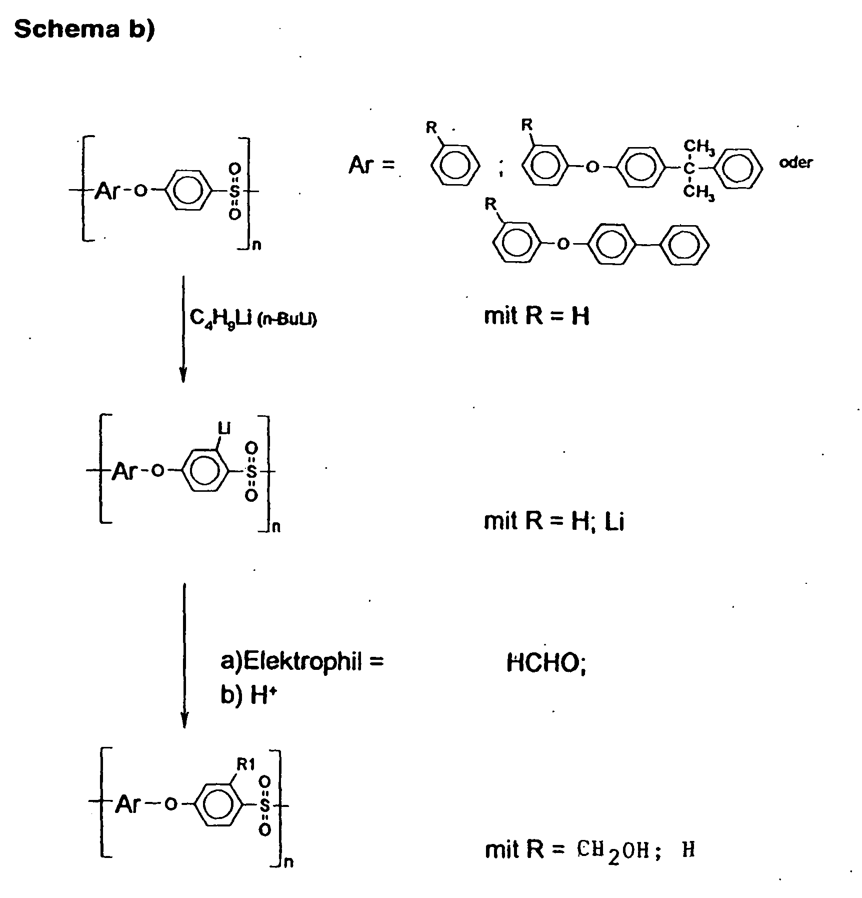

Schema b) zeigt die Methode der Metallierung der in aprotischen Lösungsmitteln gelösten oder gequollenen Polyarylsulfone mit n-Butyllithium (BuLi) bei Temperaturen zwischen -65 °C und -90 °C in Argon-Atmosphäre mit anschließender Umsetzung der carbanionisierten Polyarylsulfone mit den elektrophilen Verbindungen. Als elektrophile Verbindung wird Formaldehyd eingesetzt.

Die Verwendung von Formaldehyd führt zu hydrophilem methylenhydroxylierten Polyarylsulfon der im folgenden Schema c) dargestellten Einheiten. Die dargestellten substituierten Basispolymere sind besonders für die erfindungsgemäße Membranen geeignet. Aus den verschiedenen derivatisierten Polymeren lassen sich aufgrund ihrer Löslichkeit in Carbonsäure-N-alkylamiden und in N-Methylpyrrolidon(2) Kapillar-, Folien- oder Rohrmembranen herstellen.

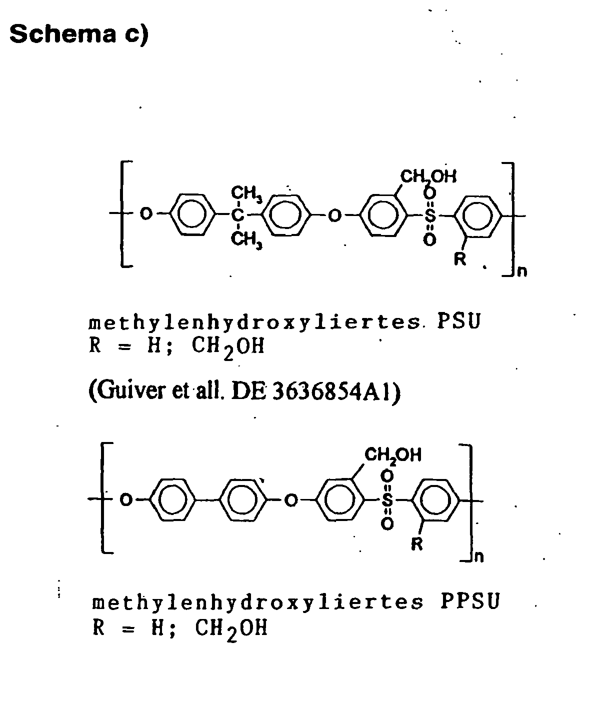

Die Substituierung erfolgt in ortho-Stellung zur Sulfongruppe an den der Sulfongruppe benachbarten Phenylengruppen, wobei vorzugsweise jede Phenylengruppe nicht mehr als einfach substituiert ist. In der Regel weisen die Polymere keine weiteren Substituenten neben der Substituierung an den Phenylengruppen auf.

Die erfindungsgemäß substituierten Polymere werden vorzugsweise in einem inerten Lösungsmittel gelöst, das mit Wasser unbegrenzt mischbar ist. Die Herstellung der Membranen kann beispielsweise nach dem Phaseninversions-Naßspinnverfahren mit Hilfe von 2- oder 3-Stoff-Naßspinndüsen durch Fällung mit Wasser erfolgen.

Die erfindungsgemäßen Membranen, insbesondere Diffusionsmembranen, aus hydrophilen aromatischen Polysulfonen sind für niedermolekulare Proteine mit Molmassen bis zu 20 000 Dalton oder höher permeabel und impermeabel für hochmolekulare Proteine mit Molmassen, die größer als 100 000 Dalton sind. Bei der Permeation bzw. Diffusion spielt im wesentlichen nur die Porengröße der Membran eine Rolle, das heißt also die molekulare Trenngrenze der Membran. Die Antriebskraft für die Permeation ist der transmembrane osmotische Druckgradient der Permeanten.

In der Regel erfolgt die Permeation ohne weiteren hydrostatischen Druck. Es ist jedoch auch möglich, die Trennung durch Anlegung eines Druckes oder eines Vakuums zu beeinflussen.

Je nach verwendeten Polymeren und Bedingungen der Membranherstellung kann die molekulare Trenngrenze der resultierenden Membran beeinflußt werden. Hierbei sind molekulare Trenngrenzen von 5 000 Dalton bis 100 000 Dalton vorgesehen. Die gewählte molekulare Trenngrenze hängt wesentlich von der geplanten Anwendung ab. Soll die Membran beispielsweise für Insulin durchgängig sein, ist eine Membran mit einer molekularen Trenngrenze von etwa 70 000 Dalton geeignet.

Bei einer bevorzugten Ausführungsform der Erfindung handelt es sich um eine Membran von extrem geringer Dicke. Hierbei beträgt die Wandstärke der Membran 20 µm bis 400 µm. Besonders bevorzugt ist eine Wandstärke von etwa 40 µm bis etwa 150 µm. Hierdurch wird erreicht, daß die Proteine ausgesprochen schnell und vollständig die Membran passieren können. Es werden also ausgesprochen kurze Diffusionswege bereitgestellt, wodurch der Stoffaustausch sehr schnell stattfindet.
Erfindungsgemäß weist die Membran eine Porosität auf, wodurch die Diffusion bzw. Permeation von Proteinen ermöglicht wird. Die Porosität der Membran beträgt 50 % bis 90 %, bezogen auf das Gesamtvolumen der Membran. Je nach gewünschter Anwendung, insbesondere gewünschter Permeationsgeschwindigkeit, kann die Porosität der Membran variiert werden. Generell nimmt die Permeationsgeschwindigkeit bei geringerer Porosität ab.

Die molekulare Trenngrenze der Membran wird wesentlich auch durch die Porengröße bestimmt. Die Porengröße bewegt sich im Durchschnitt zwischen 10 nm und 50 nm. Die Membran weist im Querschnitt, d.h. also von einer Oberfläche zur anderen Oberfläche, eine im wesentlichen nach außen hin zunehmende durchschnittliche Porengröße auf. Hierbei beträgt die Porengröße der kleineren Poren 10 nm bis 50 nm.

Neben der der erfindungsgemäßen Membran eigenen Hydrophilie kann es bevorzugt sein, daß neben der chemisch installierten Hydrophilie eine zusätzliche Absättigung von Adsorptionsstellen der Membran vorgesehen ist. Eine derartige Präadsorption der Membran führt dazu, daß die antiadsorptiven Eigenschaften der Membran noch verstärkt und optimiert werden. Außerdem kann hierdurch die Biokompatibilität der Membran verbessert werden. In einer bevorzugten Ausführungsform wird die Membran mit fötalem Kälberserum (FCS) vorinkubiert.

Durch die Vorinkubation mit beisielsweise fötalem Kälberserum werden verschiedene nichtspezifische Bindungsstellen der Membran blockiert. Hierdurch wird ein synergistischer Effekt erzielt, der eine derart behandelte erfindungsgemäße Membran für den Einsatz in der Biomedizintechnik besonders geeignet macht.

Gemäss der Erfindung wird die Membran im sterilen Zustand verpackt, beispielsweise eingeschweißt. Dies geschieht im feuchten Zustand der Membran, beispielsweise nach einer Vorinkubation mit fötalem Kälberserum. Das Einschweißen erfolgt vorzugsweise in herkömmlichen Plastikfolien. Durch das Einschweißen ist es möglich, die erfindungsgemä-ßen Membranen für einen längeren Zeitraum zu lagern und haltbar zu machen. Die Membranen werden sterilisiert, um eine möglicherweise bakterielle Kontamination zu verhindern. In einer bevorzugten Ausführungsform geschieht die Sterilisierung durch Gamma-Bestrahlung.

Die Erfindung umfaßt weiter ein Verfahren zur Herstellung der mikroporösen, hydrophilen Membran. Bei diesem Herstellungsverfahren werden Basispolymere zunächst substituiert, die substituierten Polymere werden in einem Lösungsmittel gelöst und mit mindestens einem mit dem Lösungsmittel mischbaren Fällmittel ausgefällt. Die Substituierung der Polymere wird mit Formaldehyd vorgenommen, der mindestens eine Hydroxymethylgruppe einführt. Auf diese Weise wird eine Membran gewonnen, die aus im Prinzip hydrophoben Basispolymeren besteht, die durch Substituierung, das heißt Einführung von hydrophilen Gruppen, insgesamt hydrophile Eigenschaften aufweist. Eine solche Membran ist in besonderer Weise für die Diffusion bzw. Permeation von Proteinen bestimmten Molekulargewichts geeignet, da hier die Vorteile bezüglich der Stabilität von auf hydrophoben Polymeren basierenden Membranen mit den durch die Modifikation eingeführten hydrophilen Eigenschaften verknüpft werden, wodurch eine stabile, aber dennoch extrem antiadsorptive Membran bereitgestellt werden kann.

Die durch Umsatz mit Formaldehyd erhaltene Methylenhydroxyl-Gruppe weist besonders günstige Eigenschaften hinsichtlich der Hydrophilie, verbunden mit einer optimalen Spacerlänge, also dem Abstand zwischen hydrophiler Gruppe und Basispolymer, auf.

Bezüglich der weiteren Eigenschaften des erfindungsgemäßen Verfahrens wird auf die obige Beschreibung verwiesen.

Die Modifikation der Basispolymeren erfolgt im wesentlichen nach bekannten Methoden. Vorzugsweise erfolgt dies durch direkte Metallierung (H/Li-Austausch) oder einen Halogen-Metallaustausch.

Durch die Wahl des Fällmittels kann die Verteilung der Poren, insbesondere der Poren verschiedener Größe, im Querschnitt der Membran beeinflußt werden. Durch Verwendung von Wasser als Fällmittel kann eine Membran mit asymmetrischem Aufbau hergestellt werden, die eine im Querschnitt von einer Oberfläche zur anderen Oberfläche im wesentlichen zunehmende Porengröße aufweist. Hierbei dient die Oberfläche mit kleineren Poren als eigentliche Membran. Der Abschnitt mit größer werdender Porengröße dient als Stützstruktur bzw. Matrix.

Die Herstellung von fingerstrukturierten Kapillarmembranen erfolgt vorzugsweise durch Lösen der erfindungsgemäß substituierten Polymere in einem inerten Lösungsmittel, das mit Wasser mischbar ist. Mit Hilfe von 2- oder 3-Stoff-Naßspinn-düsen können durch Fällung mit Wasser fingerstrukturierte Kapillarmembranen hergestellt werden, die von Polymerlösungsmittel befreit werden.

Durch Fällung mit Wasser, das etwa 20 % bis 60 % des inerten Polymerlösungsmittels enthält, können Kapillarmembranen mit Mischungen aus Schwamm- und Fingerstruktur hergestellt werden.

Ein bevorzugtes Verwendungsgebiet erfindungsgemäßer Membranen stellt die Biomedizintechnik dar. Vor allem im Hinblick auf die Herstellung von Bioreaktoren und biohybriden Organen, die in einen Organismus eingebracht werden, bilden die erfindungsgemäßen Membranen ein ausgesprochen geeignetes Material, da bei diesen Membranen eine lange Lebensdauer mit optimalen funktionellen Eigenschaften gekoppelt ist.

Ein weiteres Anwendungsgebiet ist der Bereich der Blutwäsche bzw. der Dialyse. Neben den eigentlichen funktionellen Eigenschaften der Membran ist auch immer die Biokompatibilität der erfindungsgemäßen Membranen von Vorteil.

Weiterhin sind die erfindungsgemäßen Membranen für eine sog. Wirkstoff-Freisetzung im Organismus in herausragender Weise geeignet. Ein Beispiel für eine derartige Wirkstoff-Freisetzung ist das biohybride Pankreas. Hierbei wird unter Verwendung der erfindungsgemäßen Membranen ein künstliches Organ in Form einer Kammer gebildet, welche zu transplantierende Inselzellen aufnimmt. Diese Inselzellen produzieren Insulin, welches aufgrund der antiadsorptiven Eigenschaften der Membran diese Kammer, deren Begrenzungen zumindest teilweise von der erfindungsgemäßen Membran gebildet werden, verlassen und in den Organismus aufgenommen werden kann.

Gemäss der Erfindung ist dieses künstliche Organ, also die Kammer, als Kapillare ausgebildet, innerhalb derer sich die Inselzellen befinden. Beispielsweise besteht das künstliche Organ aus einer zu einem Schlauch gewendelten mikroporösen Kapillar-Diffusionsmembran, welche durch ein Polyurethan-Sprühvlies stabilisiert ist. Eine solche Kapillare wird in ein Blutgefäß eingebracht. Durch transmembrane Permeation können Glucose und essentielle Nährstoffe aus dem Blutstrom in das Kapillarinnere gelangen und dadurch die Inselzellen mit den notwendigen Nährstoffen versorgen. Auf diese Weise gelangt auch Glucose in die Kammer. Die Inselzellen produzieren Insulin infolge der Glucosestimulation. Das Insulin gelangt über die erfindungsgemäße Membran aufgrund des Konzentrationsgefälles in den Blutstrom. Auch andere von den Inselzellen sezernierten niedermolekularen Proteine gelangen auf diese Weise in den Blutstrom.

Vorteilhafterweise wird die molekulare Trenngrenze der Membran in einem derartigen biohybriden Organ derart gewählt, daß Immunglobuline aus dem Blutstrom nicht in die Kapillare gelangen können. Dies wird durch eine molekulare Trenngrenze der Membran von etwa 100 000 Dalton erreicht. Auf diese Weise wird vermieden, daß die transplantierten Inselzellen innerhalb des biohybriden Organs durch das Immunsystem angegriffen werden und damit eine Abstoßungsreaktion hervorrufen.

Eine bevorzugte Form für ein biohybrides Pankreas ist die Kapillare, da bei der Kapillarform eine maximale Raumdichte bei maximaler Diffusions-Flächendichte realisiert ist. Ein bevorzugtes Kapillarlumen beträgt etwa 600 bis etwa 800 µm. Die Wandstärke beträgt vorteilhafterweise etwa 40 bis 120 µm.

Herkömmliche Membranen sind für den Einsatz in einem biohybriden Organ, insbesondere in einem biohybriden Pankreas, nicht geeignet. Das besondere Problem liegt hierin in den sehr geringen Proteinmengen, die bei den auftretenden physiologischen Konzentrationen sich in der Größenordnung von nMol/l bewegen. Bei beispielsweise Oberflächen-modifizierten hydrophoben Membranen (adsorptive Hydrophilisierung) oder Membranen aus sulfonierten Basispolymeren werden die sehr geringen Proteinmengen nahezu vollständig von der Membran adsorbiert, so daß wenig bis gar kein gewünschtes Protein, insbesondere Insulin, die Membran passieren kann. Dies zeigen Ergebnisse von Fane et.al., Desalination 53, 37, 1985. Nach eigenen Insulin-Diffusionsmessungen trifft dies auch für Kapillarmembranen aus sulfonierten PSU praktisch relevanter Substitutionsgrade (DS) bis DS 0,76 zu. Die erfindungsgemäßen Membranen sind für diese Anwendung jedoch ausgesprochen geeignet, wie die in den Beispielen gezeigten Meßergebnisse belegen.

Ein weiterer bevorzugter Anwendungsbereich der erfindungsgemäßen Membranen liegt in Bioreaktoren, beispielsweise zur Proteinfraktionierung oder zur Proteinreinigung von beispielsweise gentechnisch hergestellten Wirkstoffen.

Weiterhin können die erfindungsgemäßen Membranen auch sehr vorteilhaft im Bereich der Zellkultur eingesetzt werden.

Weiterhin ist die Verwendung der erfindungsgemäßen Membran besonders in allen Systemen geeignet, in denen die Sezernierung von Proteinen durch lebende Zellen eine Rolle spielt. Hierbei sind die Membranen Träger für die Zellen, wobei die gleichartigen oder verschiedenen Zellen entweder nur auf einer oder beiden Seiten der Membran angesiedelt sein können. Solche Systeme können zum Beispiel als Transplantate eingesetzt werden. Die Membran kann das System in Kompartimente trennen, innerhalb derer verschiedene Kreisläufe unterschiedlicher Funktion ablaufen (z.B. Blut-/Plasma- und Nährstoffkreislauf). Durch entsprechende molekulare Ausschlußgrenzen sorgt die Membran dafür, daß die durch die Membran abgegrenzten Kompartimente gegenüber dem Immunsystem isoliert sind. Dies spielt insbesondere dann eine wichtige Rolle, wenn mit Hilfe der durch die Membran gebildeten Kompartimente Zellen in einen Patienten eingebracht werden, die von einem anderen Organismus stammen.

Neben dem bereits erwähnten biohybriden Pankreas sind auch weitere Wirkstofffreisetzungs-Systeme unter Verwendung der erfindungsgemä-ßen Membran möglich. Dies können Systeme sein, die sich im Körper langsam selbst auflösen. Weiterhin sind Systeme möglich, die lebende Zellen enthalten, die beispielsweise gentechnisch so manipuliert wurden, daß sie bestimmte Wirkstoffe permanent synthetisieren und über die erfindungsgemäße Membran in den Organismus, vorzugsweise in den Blutkreislauf, freisetzen. Bei diesen Wirkstoffen handelt es sich in der Regel um Proteine, beispielsweise um Cytokine. Ein solches System ist beispielsweise eine implantierbare Kapillarmembran, die Zellen enthält, die permanent schmerzstillende Faktoren sezernieren.

Weiterhin kann unter Verwendung der erfindungsgemäßen Membran eine biohybride Leber bereitgestellt werden, die zur Therapie von Patienten im hepatischen Koma eingesetzt werden kann. Hierbei ist die Leber soweit geschädigt, daß keine ausreichende Entgiftung des Körpers mehr stattfinden kann. Die biohybride Leber übernimmt die Detoxifizierung bis beispielsweise ein Transplantat zur Verfügung steht oder die Patientenleber sich regeneriert hat.

Weiterhin kann die erfindungsgemäße Membran eingesetzt werden, um die Kommunikation verschiedener Zellarten (z.B. des Immunsystems) mittels Botenstoffen, vorzugsweise Cytokinen, zu untersuchen. In derartigen sog. Transwell-Systemen trennt die Membran zwei verschiedene Zellarten voneinander, die sich beispielsweise gegenseitig stimulieren können. Die extrem antiadsorptiven Eigenschaften der erfindungsgemäßen Membranen sind hier von besonderer Bedeutung, da nur extrem geringe Mengen der Botenstoffe ausgeschüttet werden, die von herkömmlichen Membranen adsorbiert werden würden und so eine derartige Untersuchung vollständig unmöglich machen würden.

Die beschriebenen Merkmale sowie weitere Einzelheiten der Erfindung ergeben sich aus der folgenden Beschreibung von Ausführungsbeispielen in Verbindung mit den Unteransprüchen.

### Die Figuren zeigen:

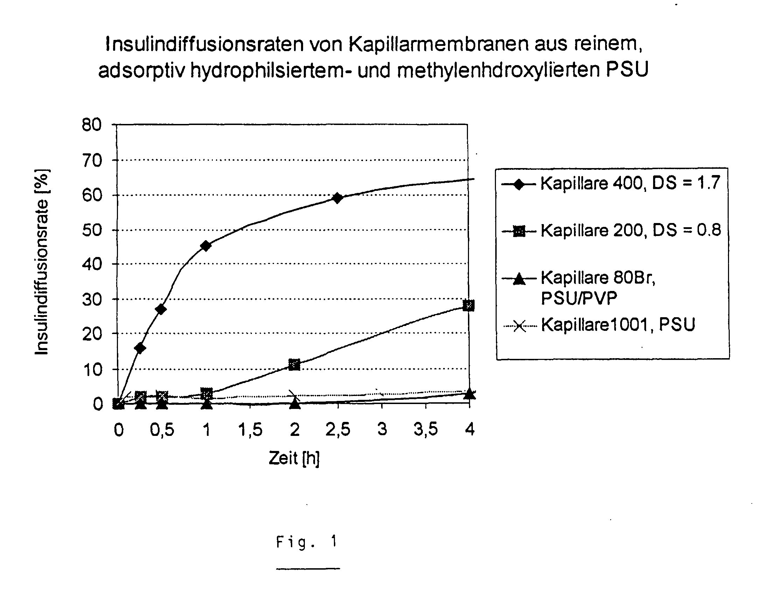

Fig. 1 Insulindiffusionsraten von Kapillarmembranen aus adsorptiv hydrophilisierten- und methylenhydroxylierten PSU. Die hydrophilen methylenhydroxylierten PSU-Membranen (Kapillare 200 und Kapillare 400) zeigen mit zunehmendem Substitutionsgrad deutlich höhere Insulin-Diffusionsraten bei kürzeren lag-Phasen (Verzögerungsphase) als die aus reinem (Kapillare 1001) oder aus mit PVP adsorptiv hydrophilisiertem (Kapillare 80Br) PSU.

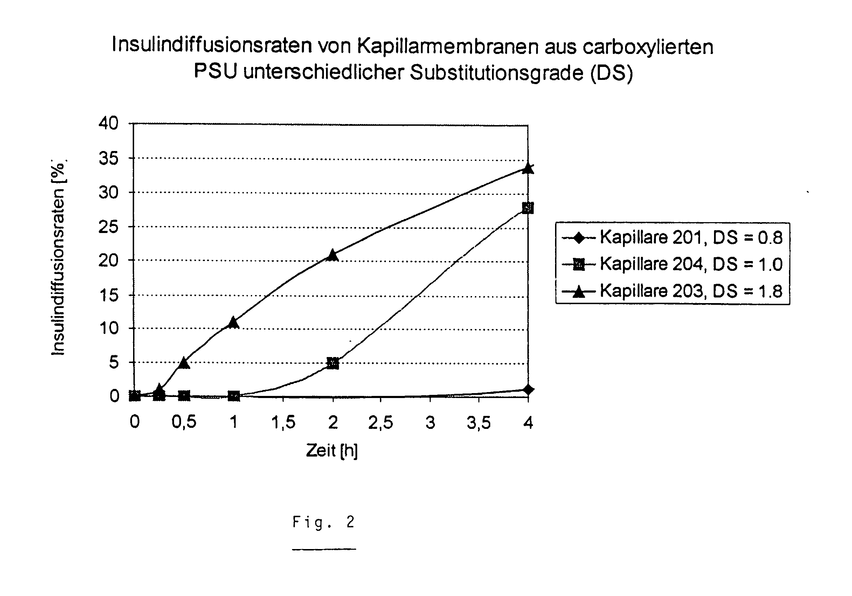

Fig. 2 Insulindiffusionsraten für Kapillarmembranen aus carboxylierten PSU unterschiedlicher Substitutionsgrade. Die carboxylierten PSU-Membranen zeigen mit zunehmendem Substitutionsgrad deutlich höhere Insulin-Diffusionsraten bei kürzeren lag-Phasen.

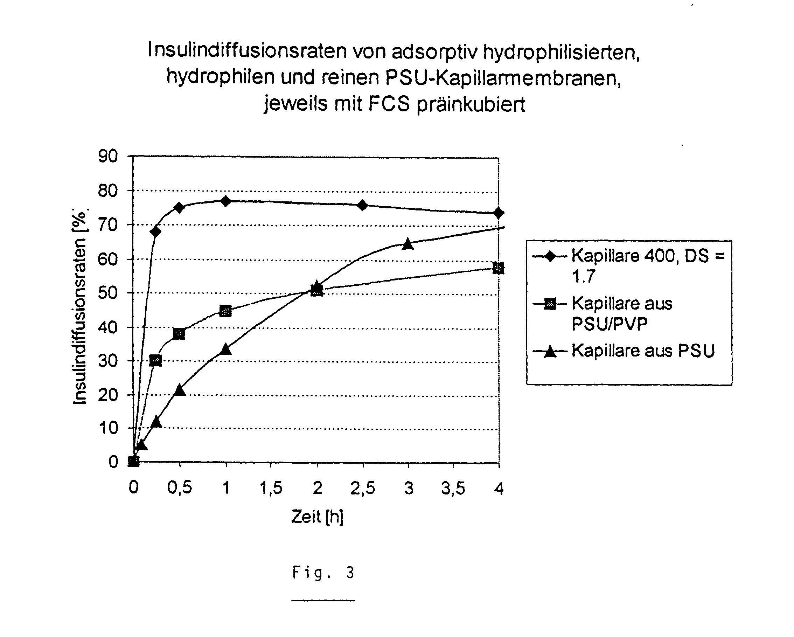

Fig. 3 Insulindiffusionsraten von adsorptiv hydrophilisierten, hydrophilen und reinen PSU-Kapil-larmembranen, jeweils mit FCS präinkubiert. Die mit FCS präinkubierten Membranen aus hydrophilem methylenhydroxyliertem (Kapillare 400), mit PVP adsorptiv hydrophylisiertem und reinem PSU zeigen im Vergleich mit nicht-präinkubierten Membranen bessere Insulindiffusionsraten und kürzere lag-Phasen. Hierbei zeigt die hydrophile Membran (Kapillare 400) bessere Insulindiffusionsraten als die Membranen aus adsorptiv hydrophilisiertem und reinem PSU.

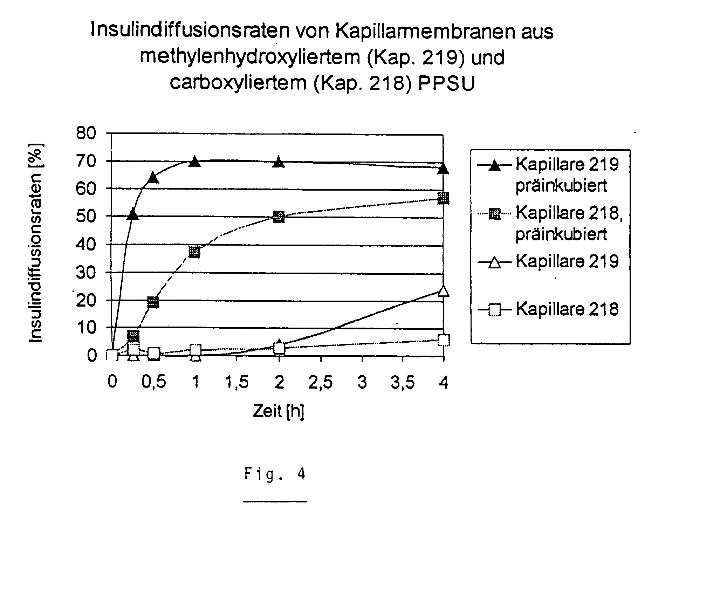

Fig. 4 Vergleich von Insulindiffusionsraten von Kapillarmembranen aus methylenhydroxyliertem und carboxyliertem PPSU mit und ohne Präinkubation. Der Vergleich zeigt, daß die mit FCS präinkubierten Membranen aus methylenhydroxyliertem (Kapillare 219) und carboxyliertem PPSU (Kapillare 218) deutlich bessere Insulin-diffusionsraten zeigen als die nicht-präinkubierten Membranen.

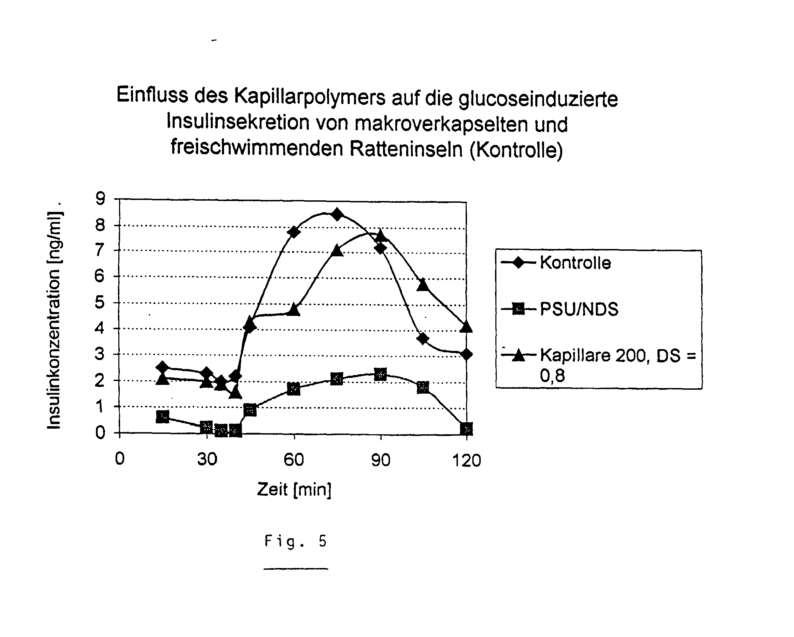

Fig. 5 Einfluß des Kapillarpolymers auf die Glucose-induzierte Insulinsekretion von makroverkapselten und frei schwimmenden Ratteninseln. Der Vergleich mit der Kontrolle (frei schwimmende Ratteninseln) zeigt, daß selbst eine relativ gering substituierte methylenhydroxylierte Membran (Kapillare 200) für Insulin wesentlich besser permeabel ist als eine nur adsorptiv hydrophilisierte (PSU/NDS).

### Beispiele

### Beispiel 1: Synthese des methylenhydroxylierten Udel^{®}-PSU

Man löst unter Argon in einem 6 1 Vierhalskolben 132 g PSU ≅ 300,8 mMol in 4 1 Tetrahydrofuran (THF). Die klare gelbliche Lösung wird auf -65 °C gekühlt und tropfenweise innerhalb von 20 min. mit 66,4 ml 10M n-Butyllithium (BuLi) ≅664 mMol versetzt. Dabei wird die Lösung erst grünlich und dann rot-braun. Bei -65 °C bis -80 °C wird während 1,5 Std. nachgerührt und dann 56 g Formaldehyd = 1700 mMol, erhalten durch Thermolyse aus Paraformaldehyd, gelöst in 1,5 1 THF, ebenfalls auf -65 °C bis -80 °C vorgekühlt, zu. Sehr langsam unter stetigem Erwärmen auf Raumtemperatur erhält man eine gelbliche, heterogene Mischung (PSU.CH₂OLi). Diese wird durch Ansäuern mit 37 %iger Salzsäure (HCl) in die Hydroxylverbindung überführt, daran erkenntlich, daß sich der Niederschlag vollständig löst. Das THF wird abdestilliert und das Polymer in 500 ml N,N-Dimethylacetamid (DMAc) aufgenommen. Die DMAc-Lösung wird in 500 ml vollentsalztes Wasser (VE-Wasser) gegossen, wobei das Polymer ausgefällt und im Labormixer zerkleinert wird. Nach dem Abfiltrieren des Polymers wird es dreimal mit kochendem VE-Wasser ausgewaschen und im Trockenschrank bei 105 °C über Nacht getrocknet.
Es wird unvernetztes methylenhydroxyliertes PSU erhalten.

### Thermolyse des Paraformaldehyds:

Ca. 60 g Paraformaldehyd werden in einem Dreihalskolben auf 160 °C erhitzt und der entstehende monomere Formaldehyd mit einem schwachen Argonstrom in auf -65 °C bis -80 °C gekühltes THF geleitet, in dem er sich löst.
Die OH-Gruppen der Hydroxymethylengruppen wurden IR-spektroskopisch nachgewiesen.

### Bestimmung des Substitutionsgrades (DS) mittels¹H-NMR-Spektroskopie:

Eine Probe des trockenen Polymers wurde in deuteriertem Dimethylsulfoxid (DMSO) gelöst und das ¹H-NMR-Spektrum aufgenommen. Durch Beziehen der Integrale der Methylengruppen auf das Integral der Methylgruppen der Isopropylideneinheit des PSU ergabe sich ein DS von 1,9.

### Beispiel 2: Variation von Beispiel 1.

Synthese und Analytik wie in Beispiel 1 beschrieben mit den Unterschieden, daß 132 g PSU = 300,8 mMol mit 24,5 ml 10M BuLi = 245 mMol umgesetzt und das metallierte PSU mit 21 g Formaldehyd = 627 mMol abgefangen wurde.
Die ¹H-NMR-spektroskopische Analyse ergab methylenhydroxylterminiertes PSU mit einem DS von 0,7.

### Beispiel 3: Synthese des carboxylierten PPSU (Vergleichsbeispiel)

Unter Argon quillt man bei Raumtemperatur in einem 2 1 Vierhalskolben 30 g PPSU ≅ 75,0 mMol, das aus seiner Lösung in DMAc mit VE-Wasser umgefällt wurde, mit 1,2 1 THF. Die Suspension wird auf -78 °C gekühlt und tropfenweise innerhalb von 20 min. mit 9 ml 10M BuLi≅ 90 mMol versetzt. Bei -78 °C wird dann während 30 min. nachgerührt und unter intensivem Rühren bei -78 °C CO₂ eingeleitet. Dabei wird die Suspension heller, bis eine gleichmäßige farblose Masse entsteht. Unter Rühren läßt man auf Raumtemperatur erwärmen. Dann wird die Suspension (PPSU-COOLi) durch Ansäuern mit 37 %iger HC1 in die Carbonsäureform überführt. Das THF wird abdestilliert und das Polymer in 200 ml DMAc aufgenommen und nach Beispiel 1 aufgearbeitet. Es wird unvernetztes carboxylterminiertes PPSU erhalten.
Die Umsetzung wurde IR- und ¹H-NMR-spektroskopisch nachgewiesen.

### Beispiel 4: Synthese des methylenhydroxylierten PPSU

Unter Argon quillt man in einem 2 l Dreihalskolben 30 g PPSU ≅ 75 mMol in 1,2 l THF. Beim Abkühlen auf -78 °C erhält man eine stark gequollene Suspension, die tropfenweise innerhalb von 20 min. mit 9 ml 10M Buli ≅ 90 mMol versetzt wird. Bei -65 °C wird 0,5 Std. nachgerührt und dann 12 g Formaldehyd (≅400 mMol), wie in Beispiel 1 beschrieben, gelöst in 0,8 1 THF, auf -65 °C vorgekühlt, zugegeben. Unter Rühren läßt man auf Raumtemperatur erwärmen und überführt die erhaltene Lithium-Verbindung mit 37 %iger HC1 in die Hydroxylform. Das THF wird abdestilliert und das Polymer in 200 ml DMAc aufgenommen und wie unter Beispiel 1 beschrieben aufgearbeitet.
Es wird unvernetztes methylenhydroxyliertes PPSU erhalten.

Die Umsetzung wurde IR- und ¹H-NMR-spektroskopisch nachgewiesen.

### Beispiel 5: Synthese des carboxylierten PES (Vergleichsbeispiel)

20 g ≅ 86,3 mMol PES wurden in 1 l über Molekularsieb 4Å getrocknetem 1,3-Dioxolan während einer Stunde bei Raumtemperatur gequollen. Nach Kühlen auf -78 °C wurden unter Rühren 10,4 ml 10M BuLi = 104 mMol zugetropft und weitere 3 Stunden nachgerührt, wobei sich die anfänglich farblose Suspension nach bräunlich-beige verfärbte. Danach wurde bei -78 °C CO₂ eingeleitet, wobei sich die Suspension deutlich aufhellte. Nach Erwärmen auf Raumtemperatur wurde mit 37 %iger HC1 angesäuert, das Dioxolan abdestilliert, der Rückstand in DMAc aufgenommen und wie unter Beispiel 1 beschrieben aufgearbeitet.
Die Umsetzung wurde IR- und ¹H-NMR-spektroskopisch nachgewiesen.

### Beispiel 6: Herstellung der Kapillarmembranen mit Fingerstruktur

Aus den vorgenannten Polymeren wurden Gießlösungen mit 20 % Polymergehalt in 75 % DMAc und 5 % LiCl hergestellt und mit 2- oder 3-Stoff-Naßspinndüsen (Eigenentwicklungen zum Teil in Zusammenarbeit mit der Firma Lechler, Metzingen, Deutschland) mit einem Fällmittel zu asymmetrischen Kapillarmembranen nach dem Phaseninversionsprinzip verarbeitet. Hierbei wurde die Polymer-Gießlösung durch eine äußere Ringdüse gefördert und mit Fällmittel, das durch eine zentrale Innendüse gefördert wurde, kontaktiert. Nach Durchlaufen einer definierten freien Fällstrecke tauchten die Kapillaren in ein äußeres Fällbad mit VE-Wasser ein zum weiteren Lösungsmittel-Fällmittel-Austausch, der durch Spülen der Kapillarmembranen über Nacht mit fließendem Leitungswasser komplettiert wurde.

Die Kapillarmembranen hatten eine innenliegende Skinschicht und eine außenliegende Stützschicht. Ihre Lumina betrugen zwischen 650 und 850 µm bei nach Maßgabe ausreichender mechanischer Stabilität extrem geringen Gesamtwandstärken zwischen 40 und 100 µm.

Durch Variation der Spinnparameter
- Verhältnis des Durchmessers der Ringdüse zum Außendurchmesser der Innendüse
- Verhältnis der Gießlösungs- zu den Fällmittelmengen
- Fällhöhe
   lassen sich Kapillarlumen und -wandstärke variieren.

### Beispiel 7: Herstellung der Kapillarmembranen mit partieller Schwammstruktur

Die Verfahrensweise war die des Beispiels 7 mit dem Unterschied, daß mit 20 bis 60 %igen Lösungen von DMAc in VE-Wasser gefällt wurde. Auf diese Weise wurden Kapillarmembranen mit zunehmendem Schwammstruktur-Anteil hergestellt, je größer der DMAc-Gehalt im Fällmittel war.

### Beispiel 8: Bestimmung der Insulin-Diffusionsraten

Kapillarmembran-Proben aus PSU, mit Polyvinylpyrrolidon (PVP) hydrolysiertem PSU und aus hydrophilen PSU wurden jeweils mit einer Lösung von 239 U/l (1.434 Mol) Insulin in Tris/HCl-Puffer, pH 7,4 befüllt, beidseitig verschlossen, in magnetgerührte Bechergläser mit je 80 ml reinem Tris/HCl-Puffer, pH 7,4 (Außenphase) eingehängt und die Diffusionsraten für Insulin als zeitabhängige Menge des permeierten Insulins in % der in der Membran vorgelegten Gesamt-Insulin-Menge immunologisch-photometrisch (ELISA[DAKO Insulin]) ermittelt.

Die Ergebnisse, in den Fign. 1 bis 4 dargestellt, belegen:
- Die hydrophilen methylenhydroxylierten PSU-Membranen zeigen mit zunehmendem DS deutlich höhere Insulin-Diffusionsraten bei kürzeren lag-Phasen als die aus reinem oder aus mit PVP adsorptiv hydrophilisiertem PSU (Fig. 1).
- Die Membranen aus carboxyliertem PSU zeigen ab einem DS von 0,8 ebenfalls höhere Insulin-Diffusionsraten und kürzere lag-Phasen als die aus reinem oder aus mit PVP adsorptiv hydrophilisiertem PSU. Ein Vergleich zeigt, daß Membranen aus methylen-hydroxyliertem PSU bessere Insulin-Diffusionsraten und kürzere lag-Phasen aufweisen als Membranen aus carboxyliertem PSU vergleichbarer Substitutionsgrade. Dies kann mit einer besseren hydrophilen Wirkung des CH₂-Spacers zwischen aromatischem Ring und OH-Gruppe beim methylenhydroxylierten PSU erklärt werden (Fign. 1 und 2).
- Bei mit FCS präinkubierten Membranen aus hydrophilem methylenhydroxyliertem (Kapillare 400), mit PVP adsorptiv hydrophilisiertem und reinem PSU wurden zwar in jedem Fall bessere Insulin-Diffusionsraten und kürzere lag-Phasen erzielt, aber mit dem qualitativen Unterschied, daß die hydrophile Membran wesentlich bessere Insulin-Diffusionsraten aufwies als die Membranen aus adsorptiv hydrophilisiertem und reinem PSU, deren Werte in obengenannter Reihenfolge innerhalb der praktisch relevanten Diffusionszeit von 30 min. zunehmend schlechter waren. Die beste Insulin-Diffusionsrate und eine extrem kurze lag-Phase von ca. 1,7 min. wurde mit der Kapillare 400 aus methylenhydroxyliertem PSU, dem Polymer mit gespacerten Hydroxylgruppen an der Polymerkette, erreicht (Fig. 3).
- Ein Vergleich der Diffusionsraten und der lag-Phasen der hydrophilen Membran Kapillare 400 mit und ohne Präinkubation mit FCS (Fign. 1 und 3) zeigt, daß die Kombination aus Substitution der Basispolymeren mit hydrophilen Gruppen, insbesondere mit Methylenhydroxylgruppen, und Präinkubation mit FCS die besten Diffusionsraten für niedermolekulare Proteine, wie Insulin, und die kürzesten lag-Phasen erreicht wurden.
- Beide Präparationen wirken auf die transmembrane Diffusion niedermolekularer Proteine synergistisch.
- In gleicher Weise wurden Kapillarmembranen aus methylenhydroxyliertem (Kapillare 219) und aus carboxyliertem (Kapillare 218) PPSU mit und ohne Präadsorption mit FCS auf Insulinpermeabilität getestet. Die Ergebnisse (Fig. 4) bestätigen die Befunde entsprechender Membranen aus PSU. Nur die Kombination aus Substitution des PPSU mit hydrophilen Gruppen und Präadsorption mit FCS führt zu optimalen Insulindiffusionsraten.

### Beispiel 9: Bestimmung der Insulin-Diffusion von in Membranen makroverkapselten Inselzellen nach Glucosestimulation in vitro:

Jeweils 50 frisch isolierte Ratten-Inselzellen in KREBS-RINGER-HEPES-Pufferlösung vom pH 7,4 mit 3 mMol/l Glucose wurden in mit FCS präinkubierte Probekapillarmembranen aus methylenhydroxyliertem (DS = 0,75) und aus mit Natriumdodecylsulfat (NDS) adsorptiv hydrophilisiertem PSU eingefüllt, beidseitig verschlossen (makroverkapselt) und in der gleichen Pufferlösung (Auβenmedium) perifundiert. Nach Stimulation zwischen Minute 30 und 45 durch Erhöhung der Glucosemenge auf 16,7 mMol/| wurde das von den Inselzellen sezernierte und transmembran diffundierte Insulin radioimmunologisch (RIA) bestimmt und mit der Insulinsezernierung im Außenmedium freischwimmender Inselzellen ohne Membranbarriere (Kontrolle) verglichen.
Die Ergebnisse, Mittelwerte aus jeweils 7 Meßreihen, dargestellt in Fig. 5 zeigen, daß selbst eine relativ gering substituierte methylenhydroxylierte Membran für Insulin wesentlich besser permeabel ist als eine nur adsorptiv hydrophilisierte.

### Beispiel 10: Bestimmung der molekularen Trenngrenze

Jeweils 3 Proben der Kapillarmembranen aus methylenhydroxyliertem PSU, DS = 1,7 wurden mit je 100 µl einer 20 %igen Albuminlösung (bovine-Fraktion V, Molgewicht 69 000 g/Mol) in 10 mmolarem Tris-HCl-Puffer, pH 7,4 befüllt und gegen 80 ml der gleichen Pufferkonzentration während 120 min. dialysiert.
In den Dialysatproben war photometrisch kein Albumin nachweisbar (Gesamtprotein-Bestimmung mit Farbreagenz Bio Rad Nr. 016953 bei 595 nm).
Die molekulare Trenngrenze der Membranen ist daher mit ≤ 69 000 anzusetzen.

## Patentansprüche

1. Mikroporöse hydrophile Membran in Form einer Kapillare aus chemisch substituiertem Polysulfon, welches kovalent gebundene Methylenhydroxylgruppen aufweist, wobei jede sich wiederholende Polymereinheit einen Substitutionsgrad (DS) von 0,5 bis 1,9 Methylenhydroxylgruppen besitzt, die Membran eine Stärke von 20 µm bis 400 µm aufweist, eine Porosität, bezogen auf das Volumen, von 50 % bis 90 % und im Querschnitt eine im wesentlichen zunehmende porengröße aufweist, wobei die Porengröße im Bereich der kleineren Poren zwischen 10 nm und 50 nm beträgt, die Membran eine molekulare Trenngrenze von 5.000 Dalton bis 100.000 Dalton aufweist und in sterilem, feuchtem Zustand verpackt ist.

2. Membran nach Anspruch 1, **dadurch gekennzeichnet, daß** die Polysulfone aromatische Polysulfone sind, insbesondere Polyphenylensulfone.

3. Membran nach einem der vorhergehenden Ansprüche, **dadurch gekennzeichnet, daß** die Membran eine Membranstärke von 40 µm bis 150 µm aufweist.

4. Membran nach einem der vorhergehenden Ansprüche, **dadurch gekennzeichnet, daß** die Membran zusätzlich adsorptiv abgesättigt ist, vorzugsweise mit fötalem Kälberserum.

5. Verfahren zur Herstellung einer mikroporösen, hydrophilen Membran nach einem der vorhergehenden Ansprüche, im wesentlichen umfassend die folgenden Schritte:
a) Substituieren von Polysulfon-Polymeren unter Einführung von Methylhydroxyl-Gruppen,
b) Lösen der Polymere in einem Lösungsmittel,
c) Formen der Membran, insbesondere durch Auftragen der Lösung auf einen Träger oder durch einen Spinnprozess,
d) Ausfällen mit mindestens einem mit dem Lösungsmittel mischbaren Fällmittel unter Bildung einer Membran mit einer im Querschnitt im wesentlichen zunehmenden Porengröße.

6. Verfahren nach Anspruch 5, **dadurch gekennzeichnet, daß** die Substitution unter Verwendung von Formaldehyd vorgenommen wird.

7. Verfahren nach einem der Ansprüche 5 oder 6, **dadurch gekennzeichnet, daß** die Polymere vor dem Substituieren aktiviert werden, vorzugsweise mit mindestens einer Lithiumverbindung.

8. Verfahren nach einem der Ansprüche 5 bis 7, **dadurch gekennzeichnet, daß** das Polymer an den der Sulfongruppe benachbarten Phenylengruppen in ortho-Stellung zu der Sulfongruppe substituiert wird, wobei vorzugsweise jede Phenylengruppe nicht mehr als einfach substituiert wird.

9. Verfahren nach einem der Ansprüche 5 bis 8, **dadurch gekennzeichnet, daß** Wasser als Fällmittel eingesetzt wird.

10. Verwendung der mikroporösen, hydrophilen Membran gemäß mindestens einem der Ansprüche 1 bis 4 zur diffusiven Trennung von Proteinen.

11. Verwendung der mikroporösen, hydrophilen Membran gemäss einem der Ansprüche 1 bis 4 zur Dialyse.

12. Verwendung der mikroporösen, hydrophilen Membran gemäß mindestens einem der Ansprüche 1 bis 4 zur Herstellung eines biohybriden Hohlorgans mit mindestens einer Kammer zur Aufnahme von Organzellen, wobei die Kammerwandungen mindestens teilweise von der Membran gebildet sind.

13. Biohybrides Hohlorgan mit mindestens einer Kammer zur Aufnahme von Organzellen, **dadurch gekennzeichnet, daß** die Kammerwandungen mindestens teilweise von einer mikroporösen, hydrophilen Membran gemäß einem der Ansprüche 1 bis 4 gebildet sind.

14. Verwendung nach Anspruch 12 **dadurch gekennzeichnet, daß** das biohybride Hohlorgan ein biohybrides Pankreas ist.

15. Verwendung der mikroporösen, hydrophilen Membran nach einem der Ansprüche 1 bis 4 zum Stoffaustausch.

## Claims

1. Microporous hydrophilic membrane in the form of a capillary of chemically substituted polysulphone which has covalently bonded methylene hydroxyl groups, wherein each repeating polymer unit has a degree of substitution (DS) of 0.5 to 1.9 methylene hydroxyl groups, the membrane has a thickness of 20 µm to 400 µm, a porosity based on the volume of 50% to 90%, and in the cross section an essentially increasing pore size, the pore size being in the range of the smaller pores between 10 nm and 50 nm, the membrane having a molecular separation limit of 5000 dalton to 100 000 dalton and being packed in the sterile moist state.

2. Membrane according to Claim 1, **characterized in that** the polysulphones are aromatic polysulphones, in particular polyphenylene sulphones.

3. Membrane according to one of the preceding claims, **characterized in that** the membrane has a membrane thickness of 40 µm to 150 µm.

4. Membrane according to one of the preceding claims, **characterized in that** the membrane is in addition saturated by adsorption, preferably with foetal calf serum.

5. Process for producing a microporous hydrophilic membrane according to one of the preceding claims, essentially comprising the following steps:
a) substitution of polysulphone polymers with introduction of methylhydroxyl groups,
b) dissolution of the polymers in a solvent,
c) forming the membrane, in particular by applying the solution to a support or by a spinning process,
d) precipitation with at least one solvent-miscible precipitant, forming a membrane having a pore size essentially increasing in the cross section.

6. Process according to Claim 5, **characterized in that** the substitution is performed using formaldehyde.

7. Process according to one of Claims 5 or 6, **characterized in that** the polymers are activated before the substitution, preferably using at least one lithium compound.

8. Process according to one of Claims 5 to 7, **characterized in that** the polymer is substituted at the phenylene groups adjacent to the sulphone group in ortho-position to the sulphone group, preferably, each phenylene group being no more than monosubstituted.

9. Process according to one of Claims 5 to 8, **characterized in that** water is used as precipitant.

10. Use of the microporous hydrophilic membrane according to at least one of Claims 1 to 4 for separation of proteins by diffusion.

11. Use of the microporous hydrophilic membrane according to one of Claims 1 to 4 for dialysis.

12. Use of the microporous hydrophilic membrane according to at least one of Claims 1 to 4 for producing a biohybrid hollow organ having at least one chamber for accommodating organ cells, the chamber walls being formed at least in part by the membrane.

13. Biohybrid hollow organ having at least one chamber for accommodating organ cells, **characterized in that** the chamber walls are formed at least in part by a microporous hydrophilic membrane according to one of Claims 1 to 4.

14. Use according to Claim 12, **characterized in that** the biohybrid hollow organ is a biohybrid pancreas.

15. Use of the microporous hydrophilic membrane according to one of Claims 1 to 4 for mass transfer.

## Revendications

1. Membrane hydrophile microporeuse sous forme d'un capillaire, constituée par une polysulfone chimiquement substituée, qui présente des groupes méthylènehydroxyle liés par covalence, chaque unité polymère qui se répète présentant un degré de substitution (DS) de 0,5 à 1,9 groupe méthylènehydroxyle, la membrane présentant une épaisseur de 20 µm à 400 µm, une porosité, par rapport au volume, de 50% à 90% et une taille de pores qui augmente essentiellement en coupe transversale, la taille des pores étant comprise entre 10 nm et 50 nm dans la zone des petits pores, la membrane présentant une limite de séparation moléculaire de 5 000 Daltons à 100 000 Daltons et étant emballée dans un état stérile, humide.

2. Membrane selon la revendication 1, **caractérisée en ce que** les polysulfones sont des polysulfones aromatiques, en particulier des polyphénylènesulfones.

3. Membrane selon l'une quelconque des revendications précédentes, **caractérisée en ce que** la membrane présente une épaisseur de 40 µm à 150 µm.

4. Membrane selon l'une quelconque des revendications précédentes, **caractérisée en ce que** la membrane est en outre saturée en adsorption, de préférence avec du sérum foetal de veau.

5. Procédé pour la production d'une membrane hydrophile microporeuse selon l'une quelconque des revendications précédentes, présentant essentiellement les étapes suivantes :
a) substitution de polymères de polysulfone avec introduction de groupes méthylènehydroxyle,
b) dissolution des polymères dans un solvant,
c) façonnage de la membrane, en particulier par application de la solution sur un support ou par un processus de filage,
d) précipitation avec au moins un agent de précipitation miscible avec le solvant avec formation d'une membrane présentant une taille de pores essentiellement croissante en coupe transversale.

6. Procédé selon la revendication 5, **caractérisé en ce que** la substitution est réalisée avec utilisation de formaldéhyde.

7. Procédé selon l'une quelconque des revendications 5 ou 6, **caractérisé en ce que** les polymères sont activés avant la substitution, de préférence par au moins un composé à base de lithium.

8. Procédé selon l'une quelconque des revendications 5 à 7, **caractérisé en ce que** le polymère est substitué sur les groupes phénylène adjacents au groupe sulfone, en position ortho par rapport au groupe sulfone, chaque groupe phénylène n'étant de préférence pas substitué plus d'une fois.

9. Procédé selon l'une quelconque des revendications 5 à 8, **caractérisé en ce qu'**on utilise de l'eau comme agent de précipitation.

10. Utilisation de la membrane hydrophile microporeuse selon au moins l'une quelconque des revendications 1 à 4 pour la séparation par diffusion de protéines.

11. Utilisation de la membrane hydrophile microporeuse selon l'une quelconque des revendications 1 à 4 pour la dialyse.

12. Utilisation de la membrane hydrophile microporeuse selon au moins l'une quelconque des revendications 1 à 4 pour la production d'un organe creux biohybride présentant au moins une chambre destinée à recevoir des cellules d'un organe, les parois de la chambre étant constituées au moins partiellement par la membrane.

13. Organe creux biohybride présentant au moins une chambre destinée à recevoir des cellules d'un organe, **caractérisé en ce que** les parois de la chambre sont constituées au moins partiellement par une membrane hydrophile microporeuse selon l'une quelconque des revendications 1 à 4.

14. Utilisation selon la revendication 12, **caractérisée en ce que** l'organe creux biohybride est un pancréas biohybride.

15. Utilisation de la membrane hydrophile microporeuse selon l'une quelconque des revendications 1 à 4 pour l'échange de substances.
